# FASCICULE DE BREVET EUROPEEN

(11) **EP 4 007 630 B1**
(45) Date de publication et mention de la délivrance du brevet: **03.09.2025**
(21) Numéro de dépôt: 20754793.6
(22) Date de dépôt: 30.07.2020
(51) Int. Cl.: A61N 1/36, A61N 1/04

(54) **PROGRAMMATION D'UN DISPOSITIF D'ELECTROSTIMULATION TRANSCUTANEE**
PROGRAMMIERUNG EINER TRANSKUTANEN ELEKTROSTIMULATIONSVORRICHTUNG
PROGRAMMING OF A TRANSCUTANEOUS ELECTROSTIMULATION DEVICE

(30) Priorité: 02.08.2019 FR 1908885
(43) Date de publication de la demande: 08.06.2022
(73) Titulaire: URGO RECHERCHE INNOVATION ET DEVELOPPEMENT, 21300 Chenôve (FR)
(72) Inventeur: SAVELII, Inna, 21490 Varois et Chaignot (FR); GUY, Martine, Paulette, Jacqueline, 21121 Daix (FR)
(74) Mandataire: Bandpay & Greuter
(86) Numéro de dépôt international: PCT/FR2020/051405
(87) Numéro de publication internationale: WO 2021/023930

(56) Documents cités:
- US-A1- 2011 288 610
- US-A1- 2013 304 176
- US-A1- 2014 249 601
- US-A1- 2015 174 402
- US-A1- 2016 346 543
- US-A1- 2017 128 722

## Description

### DOMAINE DE L'INVENTION

La présente invention concerne le domaine technique des dispositifs d'électrostimulation transcutanée.

### ARRIERE-PLAN TECHNIQUE

Le message de la douleur est transmis depuis la zone douloureuse jusqu'au cerveau par l'intermédiaire de fibres nociceptives. On connait deux types de fibres nociceptives. Le premier type est les fibres Aδ (A delta) qui sont impliquées dans une transmission rapide de l'information jusqu'au cerveau par exemple dans le cas de douleurs immédiates, intenses et très brèves. Le deuxième type de fibres est les fibres C qui sont les plus nombreuses et qui sont impliquées pour une transmission lente et durable de l'information par exemple pour une douleur persistante et diffuse.

La neurostimulation électrique transcutanée procure le soulagement de la douleur à l'aide d'un courant électrique d'une intensité qui est spécifiquement adaptée. Cette méthode non médicamenteuse est utilisée quotidiennement dans les centres antidouleur. La neurostimulation électrique transcutanée réduit ou supprime la douleur sur l'ensemble des tissus musculaires et articulaires douloureux, mais ne traite pas la cause de la douleur. Le courant électrique est transmis par des électrodes placées à la surface de la peau. Afin de faciliter le circuit électrique, un gel est généralement utilisé entre l'électrode et la peau. Le courant électrique crée une excitation électrique des nerfs. La douleur est atténuée grâce à deux actions. Tout d'abord une action immédiate obtenue grâce à la diminution de la transmission de la douleur vers la moelle épinière, autrement dit un « effet porte » est réalisé par le dispositif d'électrostimulation transcutanée. Il y a ensuite une action durable qui est causée par l'augmentation de la sécrétion des endorphines, hormones antidouleur naturelles sécrétées par le cerveau.

La neurostimulation électrique transcutanée est donc une vraie alternative aux antalgiques qui a été prouvée de manière scientifique par le corps médical. Elle offre en outre une atténuation de la douleur immédiate et/ou durable. De plus, c'est une solution ayant très peu d'effets secondaires, contrairement aux effets indésirables des antalgiques et anti-inflammatoires les plus communs tels que des effets secondaires digestifs et/ou cardiovasculaires.

Cependant, le réglage d'un dispositif d'électrostimulation transcutanée peut s'avérer être complexe pour le grand public car 1e nombre de combinaisons des paramètres du courant électrique est très important (théoriquement infini). De plus, seul un nombre restreint de ces combinaisons offre une efficacité thérapeutique optimale. En outre, l'intensité de la douleur peut nécessiter ou pas un soulagement rapide qui va dépendre des caractéristiques de la neurostimulation électrique. De plus, le type de douleur (musculaire, articulaire...) et l'emplacement de la douleur (ventre, tête...) nécessitent également des paramètres particuliers du courant électrique pour une plus grande efficacité.

Le document US 2014/249601 A1 décrit des procédés et dispositifs pour fournir une électrothérapie et une stimulation électrique non invasives. Le document US 2016/346543 A1 décrit un système pour une stimulation électrique transcutanée du nerf ou du muscle. Le document US 2011/288610 A1 décrit un dispositif mobile d'autostimulation transcrânienne et un procédé de commande et de régulation du dispositif. Le document US 2013/304176 A1 décrit un bandage avec un dispositif intégré, sans fil, de stimulation électrique transcutanée des neurones (TENS), et son procédé d'utilisation. Le document US 2017/128722 A1 décrit un dispositif portable pour traiter la dysménorrhée chez un patient.

Il n'existe donc pas actuellement de méthode simple de réglage d'un dispositif de neurostimulation électrique transcutanée qui soit susceptible de s'adapter à différentes situations de douleurs.

### RESUME DE L'INVENTION

La présente invention propose un procédé de réglage d'un dispositif d'électrostimulation transcutanée tel que défini par la revendication 1.

Un tel procédé améliore l'efficacité, l'utilisation et la sureté d'utilisation d'un dispositif d'électrostimulation transcutanée. En effet, le dispositif comprend une mémoire dans laquelle un programme est stocké. Ce programme est associé à au moins trois des paramètres principaux du courant électrique qui va être envoyé sur la zone de douleur, et chacun de ces paramètres est associé à une valeur. Ces paramètres et leurs valeurs respectives définissent les conditions nécessaires pour obtenir une configuration du courant électrique qui agisse sur la douleur sans qu'une intervention de l'utilisateur (par exemple une configuration manuelle) ne soit nécessaire. De plus, le procédé de réglage d'un dispositif d'électrostimulation transcutanée selon l'invention permet à un utilisateur d'utiliser tout seul le dispositif pour des parties du corps plus difficiles d'accès car l'appel d'un programme ne nécessite pas ou peu d'action de la part de l'utilisateur ; il est donc possible pour un utilisateur seul de régler le dispositif, même si ce dernier est placé sur des zones du corps moins bien accessibles, par exemple l'épaule.

Selon différents modes de réalisation, toute combinaison d'au moins l'une des caractéristiques suivantes peut être implémentée :
- sélectionner un programme comprend en outre l'opération consistant à sélectionner, par action utilisateur sur un premier organe de sélection, une nouvelle valeur d'au moins un paramètre du courant électrique délivré par l'électrode ;
- la nouvelle valeur sélectionnée du au moins un paramètre est une valeur d'intensité d'un courant électrique ;
- sélectionner un programme comprend en outre l'opération consistant à sélectionner séquentiellement, par action utilisateur sur un deuxième organe de sélection, un programme parmi au moins deux programmes stockés sur la mémoire ;
- une opération de sélection comprend en outre émettre, par un dispositif d'émission de lumière, un signal lumineux pour chaque nouvelle sélection ;
- une des opérations consistant à sélectionner un programme comprend en outre émettre, par un dispositif d'émission de son, un son qui identifie de manière unique le programme sélectionné ;
- la mémoire stockant un premier programme pour lequel la largeur des impulsions du courant électrique délivré par l'électrode est comprise en 50 et 500 microsecondes bornes incluses, de préférence entre 150 et 250 microsecondes bornes incluses, la fréquence des impulsions du courant électrique délivré par l'électrode est comprise entre 60 et 140 hertz bornes incluses, de préférence entre 80 et 120 hertz bornes incluses ;
- la mémoire stockant un deuxième programme pour lequel la largeur des impulsions du courant électrique est comprise en 50 et 500 microsecondes bornes incluses, de préférence entre 150 et 250 microsecondes bornes incluses, la fréquence des impulsions du courant électrique est comprise entre 2 et 8 hertz bornes incluses, de préférence entre 4 et 6 hertz bornes incluses ;
- les impulsions du courant électrique délivré par l'électrode sont générées en continu ;
- la mémoire stockant un troisième programme pour lequel la largeur des impulsions du courant électrique est comprise en 50 et 500 microsecondes bornes incluses, de préférence entre 150 et 250 microsecondes bornes incluses, la fréquence des impulsions du courant électrique délivré par l'électrode comprend : une première fréquence d'impulsion du courant électrique comprise entre 60 et 140 hertz bornes incluses, de préférence entre 80 et 120 hertz bornes incluses ; et une deuxième fréquence d'impulsion du courant électrique comprise entre 2 et 8 hertz bornes incluses, de préférence entre 4 et 6 hertz bornes incluses ; une première durée de génération des impulsions selon la première fréquence et une deuxième durée de génération des impulsions selon la deuxième fréquence, la première durée et la deuxième durée étant sensiblement égales ;
- la mémoire stockant un quatrième programme pour lequel la fréquence des impulsions du courant électrique délivré par l'électrode est comprise entre 1 et 150 hertz bornes incluses, la fréquence des impulsions augmentant et diminuant en fonction du temps afin de former un signal triangle ;
- la largeur des impulsions du courant électrique délivré par l'électrode est comprise en 50 et 500 microsecondes bornes incluses, de préférence entre 50 et 250 microsecondes bornes incluses, la largueur de l'impulsion diminuant pour chaque augmentation de la fréquence des impulsions et augmentant pour chaque diminution de la fréquence des impulsions ;
- les impulsions du courant électrique délivré par l'électrode sont générées par cycles discontinus ;
- la mémoire stocke un cinquième programme qui exécute successivement les premier, deuxième, troisième et quatrième programmes de manière répétée, aucun signal électrique n'étant émis entre chaque répétition afin que chaque exécution successive des trois programmes ait une durée comprise entre 45 et 75 secondes, de préférence sensiblement égale à 60 secondes ;
- la valeur de tension du courant électrique est comprise entre 3,5 et 25 volts, bornes incluses.

La présente invention propose également un programme d'ordinateur tel que défini par la revendication 15, comprenant des instructions de code de programme pour l'exécution des étapes du procédé selon l'invention lorsque ledit programme est exécuté sur une unité de commande du dispositif d'électrostimulation transcutanée. La présente invention propose également un dispositif d'électrostimulation transcutanée, tel que défini par la revendication 16.

Le dispositif peut en outre comprendre :
- la batterie, l'unité de commande et la mémoire sont agencées dans un boîtier comprenant un fond formant une surface ;
- l'électrode est disposée sur un matériau souple qui est sensiblement dans le même plan que le fond du boîtier.

### BREVE DESCRIPTION DES FIGURES

Des modes de réalisation de l'invention vont être maintenant décrits au moyen d'exemples non-limitatifs et en référence aux figures, où :
FIG. 1 est un exemple de vue de dessus d'un dispositif selon l'invention ;
FIG. 2 est un exemple de vue de dessous du dispositif de la FIG. 1 ;
FIG. 3 est un exemple de vue de côté du dispositif de la FIG. 1 ;
FIG. 4 est un deuxième exemple de vue de dessus d'un dispositif selon l'invention ;
FIG. 5 est un troisième exemple de vue de dessus d'un dispositif selon l'invention ;
FIG. 6 est un quatrième exemple de vue de dessus d'un dispositif selon l'invention ;
FIG. 7 est une représentation générique de l'intensité en fonction du temps d'impulsions de courant ;
FIG. 8 est une représentation de l'intensité en fonction du temps d'un premier exemple de programme d'impulsions de courant ;
FIG. 9 est une représentation de l'intensité en fonction du temps d'un deuxième exemple de programme de courant ;
FIG. 10 est une représentation de la fréquence en fonction du temps d'un troisième exemple de programme d'impulsions de courant ;
FIG. 11 est une représentation de l'intensité en fonction du temps de l'exemple de la FIG 10 ;
FIG. 12 est une représentation de la fréquence en fonction du temps d'un quatrième exemple de programme d'impulsions de courant ;
FIG. 13 est un tableau présentant un exemple des paramètres du programme de courant de la FIG. 12 ;
FIG. 14 est une représentation de l'intensité en fonction du temps de l'exemple de la FIG. 12 ;
FIG. 15 est un exemple schématique d'un dispositif selon l'invention ;
FIG. 16 est un exemple d'un procédé de programmation selon l'invention.

### DESCRIPTION DE MODES DE REALISATION

La présente invention concerne le réglage ou encore la programmation d'un dispositif d'électrostimulation transcutanée. Un tel dispositif permet de soulager immédiatement et/ou durablement une douleur pouvant être, mais pas limitée à, articulaire, musculaire, ... à l'aide d'un courant électrique d'une intensité et d'une fréquence adaptée.

Les modes de réalisation décrits ci-après ne doivent pas être considérés comme limitatifs au regard de l'invention qui est définie par les revendications annexées.

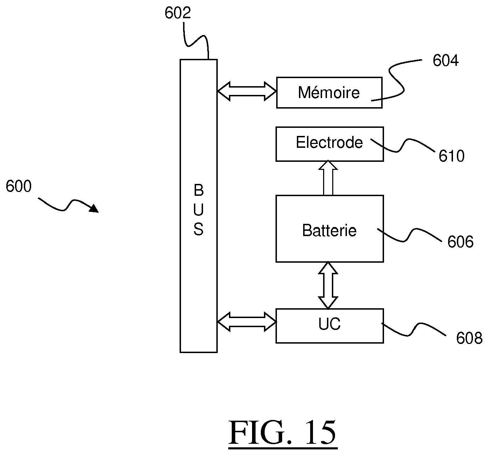

En référence à la FIG. 15, il est discuté un exemple schématique d'un dispositif 600 d'électrostimulation transcutanée. Le dispositif comprend au moins une électrode 610 qui est un dispositif conducteur d'électricité qui permet d'amener de l'énergie électrique au niveau de la peau de la partie du corps sur laquelle elle est disposée. L'énergie électrique est typiquement un courant électrique de type courant continu. Le courant continu peut être dispensé de manière discontinu. L'électrode comprend généralement une première face conductrice d'électricité, et une deuxième face qui est isolante afin de prévenir tout contact électrique entre cette deuxième face et un autre objet ou corps ou partie de corps.

Dans certains modes de réalisation, l'électrode peut comprendre un gel conducteur qui facilite la transmission de l'énergie électrique entre l'électrode et la peau, et évite des irritations cutanées au niveau de la peau. Le gel conducteur est donc déposé sur la face conductrice de l'électrode.

Dans certains modes de réalisation, le gel conducteur peut être un gel adhésif qui est déposé sur l'électrode avant que le dispositif ne soit utilisé. Le gel adhésif peut être disposé entre deux feuilles (ou encore films de protection) qui le maintienne et le protège avant son applications sur l'électrode. Les deux feuilles peuvent être en matière plastique. Lorsque le gel est disposé sur l'électrode, une des deux feuilles peut être retirée, libérant ainsi l'une des faces du gel adhésif qui va être mise au contact de l'électrode. Les propriétés adhésives du gel le maintiennent sur l'électrode. La deuxième feuille est ensuite retirée, de sorte que l'électrode présente sur sa face conductrice le gel adhésif. Lorsque le dispositif est déposé sur la zone du corps à traiter, il peut être maintenu fixe sur le corps grâce aux propriétés adhésives du gel adhésif. Lorsque le dispositif n'est pas utilisé, le gel adhésif qui est toujours sur l'électrode peut être protégé en replaçant dessus un des films de protection.

Le dispositif 600 comprend de plus une batterie 606. La batterie est un élément électrochimique dans lequel de l'énergie chimique est convertie en énergie électrique. La batterie est rechargeable, c'est-à-dire qu'il est possible d'y stocker à nouveau de l'énergie électrique, par exemple en branchant la batterie à une source externe d'électricité. La batterie rechargeable peut être de toute technologie telle que, mais n'est pas limitée à, lithium ion, lithium polymer, ... La batterie rechargeable est connectée à l'électrode, c'est-à-dire que l'énergie électrique produite par la batterie peut être transmise à l'électrode.

La batterie produit un courant de type continu et a donc une borne de sortie positive et une borne de sortie négative. L'électrode 610 se décompose donc en deux sous électrodes : une électrode positive qui est connectée à la borne positive de la batterie et une électrode négative qui est connectée à la borne négative de la batterie. Lorsque l'électrode est déposée sur la zone de peau à traiter, le courant électrique passe donc de l'électrode négative vers l'électrode positive.

Toujours en référence à la FIG. 15, le dispositif 600 d'électrostimulation transcutanée comprend également une unité de commande (UC) 608 de la batterie. L'unité de commande a pour fonction de configurer (on peut également dire conformer) le courant électrique fourni par la batterie afin qu'il ait la forme recherchée. L'énergie électrique produite par la batterie est donc transformée en un signal électrique qui est transmis à l'électrode, et cette dernière délivre ce signal électrique à la zone du corps sur laquelle l'électrode (et donc le dispositif) est appliquée. L'unité de commande est programmable, c'est-à-dire qu'elle peut être configurée pour produire différents signaux électriques à partir de l'énergie fournie par la batterie. L'unité de commande est donc semblable à une unité de calcul.

Le dispositif 600 d'électrostimulation transcutanée comprend également une mémoire qui est couplée avec l'unité de commande. Dans l'exemple de la FIG. 15, l'unité de commande et la mémoire sont couplées via un BUS 602, étant entendu qu'ils peuvent être couplés (c'est-à-dire mis en relation) par n'importe quel moyen. La mémoire est une mémoire permettant de stocker les instructions et les données nécessaires au fonctionnement d'un programme d'ordinateur. La mémoire peut-être, mais n'est pas limitée à, une mémoire non volatile, incluant par exemple des mémoires semi-conducteurs tels que des EPROM, EEPROM, mémoire flash. Tous les éléments 602, 604, 606, 608 peuvent être suppléés par ou incorporés dans, des ASICs (acronyme anglais de « application-specific integrated circuits »).

Le programme d'ordinateur peut comprendre des instructions exécutables par l'unité de commande 608. Les instructions comprennent des moyens pour amener le système à exécuter le procédé selon l'invention. Le programme peut être enregistrable sur n'importe quel support de stockage de données, y compris la mémoire 604. Le programme peut, par exemple, être mis en œuvre dans des circuits électroniques numériques, ou dans du matériel informatique, des micrologiciels, des logiciels ou des combinaisons de ceux-ci. Le programme peut être mis en œuvre sous la forme d'un appareil, par exemple un produit incorporé de manière tangible dans un dispositif de stockage lisible par machine pour une exécution par un processeur programmable. Les étapes du procédé selon l'invention peuvent être exécutées par un processeur programmable exécutant un programme d'instructions pour exécuter des fonctions du procédé en opérant sur des données d'entrée et en générant une sortie. L'unité de commande peut être ou peut comprendre le processeur programmable. Le processeur peut ainsi être programmé et couplé pour recevoir des données et des instructions, pour transmettre des données et des instructions à un système de stockage de données, à au moins un dispositif d'entrée et à au moins un dispositif de sortie. Le programme d'ordinateur peut être implémenté dans un langage de programmation procédural ou orienté objet de haut niveau, ou en langage assembleur ou machine si nécessaire. Dans tous les cas, le langage peut être un langage compilé ou interprété. Le programme peut être un programme d'installation complet ou un programme de mise à jour. L'application du programme sur le système entraîne dans tous les cas des instructions pour l'exécution de la méthode.

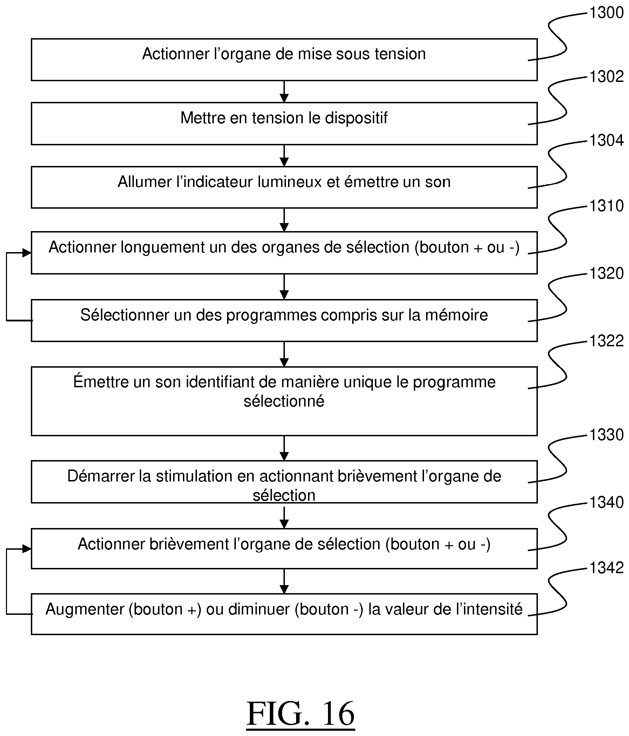

En référence à la FIG. 16, il est maintenant discuté des exemples du procédé de réglage du dispositif d'électrostimulation transcutanée. Les différents exemples peuvent se combiner entre eux, les combinaisons n'étant pas limitées à celle représentée sur la FIG. 16.

Dans des modes de réalisation selon l'invention, le procédé peut comprendre une opération de mise sous tension du dispositif d'électrostimulation transcutanée. La mise sous tension signifie que l'unité de commande est apte à recevoir des instructions d'un programme d'ordinateur stocké sur la mémoire, ou encore une instruction d'un utilisateur.

Dans des modes de réalisation selon l'invention, le dispositif d'électrostimulation transcutanée peut comprendre un organe de mise sous tension qui en réponse à une action utilisateur (1300) met le dispositif sous tension (1302). L'organe peut être un interrupteur, par exemple un bouton, sur lequel l'utilisateur appuie, par exemple, pendant une durée prédéterminée.

Dans des exemples, le dispositif peut comprendre un dispositif d'émission de son, par exemple un haut-parleur ou encore un buzzer. Après que l'utilisateur ait appuyé sur l'organe de mise sous tension, un son est émis (1304), par exemple un bip sonore, confirmant que le dispositif est bien sous tension.

Dans des exemples, le dispositif peut comprendre un dispositif d'émission de lumière, par exemple une diode électroluminescente (DEL). Après que l'utilisateur ait appuyé sur l'organe de mise sous tension, un signal lumineux est émis (1304). Le signal lumineux peut être bref, par exemple de l'ordre de quelques secondes, ou au contraire le signal lumineux peut être constamment allumé tant que le dispositif est sous tension.

Ensuite, une sélection d'un programme est réalisée. La sélection est réalisée dans la mémoire du dispositif, c'est-à-dire que le programme est stocké sur la mémoire. La sélection signifie qu'il y a identification du programme dans la mémoire, et que l'unité de commande peut accéder au programme, par exemple pour son exécution. Le programme est une liste de plusieurs paramètres du courant électrique à délivrer à l'électrode (et donc en conséquent à délivrer par l'électrode) dans laquelle chaque paramètre est associé à une valeur. Le courant électrique délivré par l'unité de commande à l'électrode est une succession d'impulsions de courant, c'est-à-dire que le courant est à l'état variable. Les impulsions sont du type signaux rectangulaires. La variation de l'intensité du courant au cours du temps produit les impulsions, et une impulsion est une variation de courte durée d'une grandeur physique du courant avec retour à l'état initial. Les paramètres du courant représentent des caractéristiques physiques de ce dernier qui peuvent varier.

L'un de ces paramètres est la tension des impulsions du courant. Sa valeur est exprimée en volts. On comprend qu'il est équivalent de parler d'intensité des impulsions du courant.

Un autre de ces paramètres est la durée des impulsions électriques. La durée des impulsions représente le temps durant lequel une tension est émise ; par exemple, pour un signal rectangulaire, il s'agit du temps pendant lequel le signal reste haut. Sa valeur peut être exprimée en seconde ou en sous-multiples.

Le troisième de ces paramètres est la fréquence des impulsions du courant électrique. La fréquence représente le nombre d'impulsions qui sont émises par seconde. Sa valeur s'exprime en Hertz.

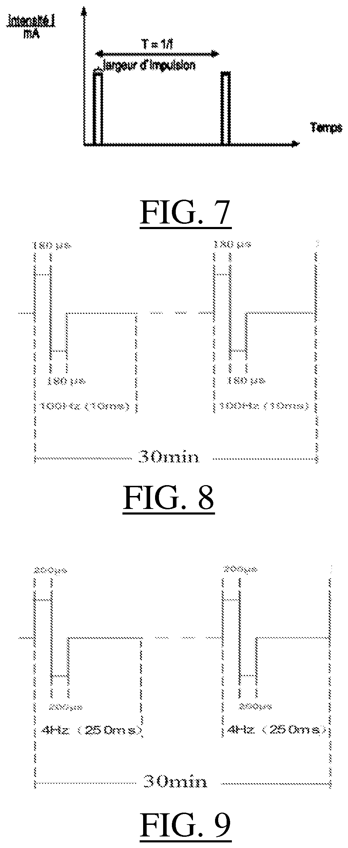

On comprendra que ces valeurs de paramètres sont particulièrement adaptées pour caractériser des impulsions électriques de type rectangulaires pouvant être utilisées pour l'électrostimulation transcutanée. La FIG. 7 illustre un exemple générique d'impulsions électriques rectangulaires pouvant être programmée. Les deux impulsions 70, 72 ont une hauteur qui représente une intensité du courant. Leurs largeurs d'impulsion sont identiques. Le temps T qui s'écoule entre deux fronts montants de deux impulsions successives est la périodicité entre deux impulsions. Dans l'exemple de la FIG. 7, les impulsions sont unidirectionnelles, c'est-à-dire que le courant est polarisé. Dans des exemples de programmes qui vont être décrits ci-dessous, les impulsions peuvent être bidirectionnelles, c'est-à-dire que le courant est dépolarisé et donc que les pôles négatif et positif s'inversent à chaque impulsion.

Dans des exemples, la mémoire du dispositif peut stocker un premier programme schématiquement représenté sur la FIG. 8, dans lequel la valeur de tension du courant électrique est comprise entre 3,5 et 17 volts bornes incluses. On note que sur la FIG. 8 l'intensité des impulsions est représentée en fonction du temps. Les impulsions sont bidirectionnelles, les valeurs de tensions sont donc données en valeur absolue. La largeur des impulsions du courant électrique est comprise en 50 et 500 microsecondes bornes incluses, de préférence entre 150 et 250 microsecondes bornes incluses. Sur la FIG. 8, toutes les impulsions ont une largeur sensiblement égale avec une valeur correspondante de 180 microsecondes. La fréquence des impulsions est comprise entre 60 et 140 hertz bornes incluses, de préférence entre 80 et 120 hertz bornes incluses. Sur la FIG. 8, la fréquence est de 100Hz. Le premier programme est une simulation de fréquence élevée. Il est adapté pour stimuler en continu les fibres nerveuses du type Aα et Aβ pour réduire le message transmis par les fibres nerveuses de la douleur Aδ et C. Ce premier programme produit donc un soulagement rapide, mais peu durable. De plus, l'utilisateur peut faire varier la valeur de l'intensité du courant électrique jusqu'à ce qu'il trouve une valeur qui lui convienne, c'est-à-dire une valeur à laquelle la zone traitée répond, ou en d'autres termes une valeur d'intensité pour laquelle l'utilisateur est soulagé.

Dans des exemples, la mémoire du dispositif peut stocker un deuxième programme schématiquement représenté sur la FIG. 9, dans lequel la valeur de tension du courant électrique est comprise entre 7 et 25 volts bornes incluses. L'intensité des impulsions est représentée en fonction du temps sur la FIG. 9. Les impulsions sont aussi bidirectionnelles. La largeur des impulsions du courant électrique est comprise en 50 et 500 microsecondes bornes incluses, de préférence entre 150 et 250 microsecondes bornes incluses. Sur la FIG. 9, toutes les impulsions ont une largeur sensiblement égale avec une valeur correspondante de 200 microsecondes. La fréquence des impulsions est comprise entre 2 et 8 hertz bornes incluses, de préférence entre 4 et 6 hertz bornes incluses. Sur la FIG. 9, la fréquence est de 4Hz. Le deuxième programme est une simulation de fréquence basse. Il est adapté pour stimuler la sécrétion d'endorphines par le cerveau, ce qui permet de soulager de façon retardée, mais durable, la douleur. La tension du courant peut être sélectionnée aux alentours de la borne supérieure mentionnée précédemment, par exemple entre 20 et 25 volts afin d'améliorer les effets susmentionnés.

Dans des exemples, les impulsions du premier programme et du deuxième programme sont générées en continue. En d'autres termes, il n'y a pas de cycles.

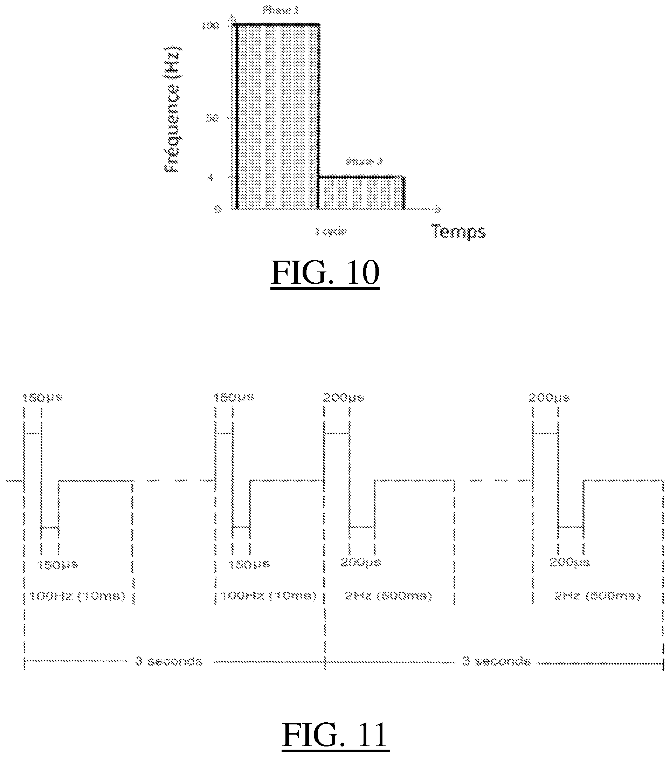

Dans des exemples, la mémoire du dispositif peut stocker un troisième programme schématiquement représenté sur la FIG. 10. Ce troisième programme comprend deux phases successives : une première phase où les impulsions sont générées à haute fréquence et une deuxième où les impulsions sont générées à basse fréquence. La fréquence des impulsions de la première phase peut être comprise entre 60 et 140 hertz bornes incluses, de préférence entre 80 et 120 hertz bornes incluses. La fréquence des impulsions de la 2ème phase peut être comprise entre 2 et 8 hertz bornes incluses, de préférence entre 4 et 6 hertz bornes incluses. La FIG. 10 illustre ces deux phases avec une première phase durant laquelle les impulsions sont générées à la fréquence de 100Hz et une deuxième phase durant laquelle les impulsions sont générées à une fréquence de 4 hertz. La largeur des impulsions du courant électrique peut être comprise entre 50 et 500 microsecondes bornes incluses, de préférence entre 130 et 220 microsecondes bornes incluses. Sur la FIG. 11, les impulsions sont représentées avec une largeur de 150 microsecondes pour la première phase et de 200 microsecondes pour la deuxième phase. Dans l'exemple de la FIG. 11, la durée de la première et de la deuxième phase sont identiques, étant entendu qu'elles peuvent être de durées différentes. La première et la deuxième phase forment un cycle. Dans des exemples les cycles peuvent être générés continuellement. Dans des exemples, et de préférence, les cycles sont discontinus avec une pause entre chaque cycle d'une durée pouvant être comprise par exemple entre 1 et 3 secondes. Dans l'exemple de la FIG. 11, un cycle comprenant la première et la deuxième phase a une durée de 6 secondes, et une pause de 1 seconde sépare chaque cycle généré. La valeur de tension du courant électrique peut être comprise entre 3,5 et 24 volts bornes incluses. On note que sur la FIG. 11 l'intensité des impulsions est représentée en fonction du temps et que les impulsions sont bidirectionnelles. Le troisième programme ressemble donc à une combinaison du premier programme (fréquence élevée, faible intensité) et du deuxième programme (fréquence faible, haute intensité) qui est générée de manière discontinue. Le soulagement apporté par le troisième programme est immédiat et durable.

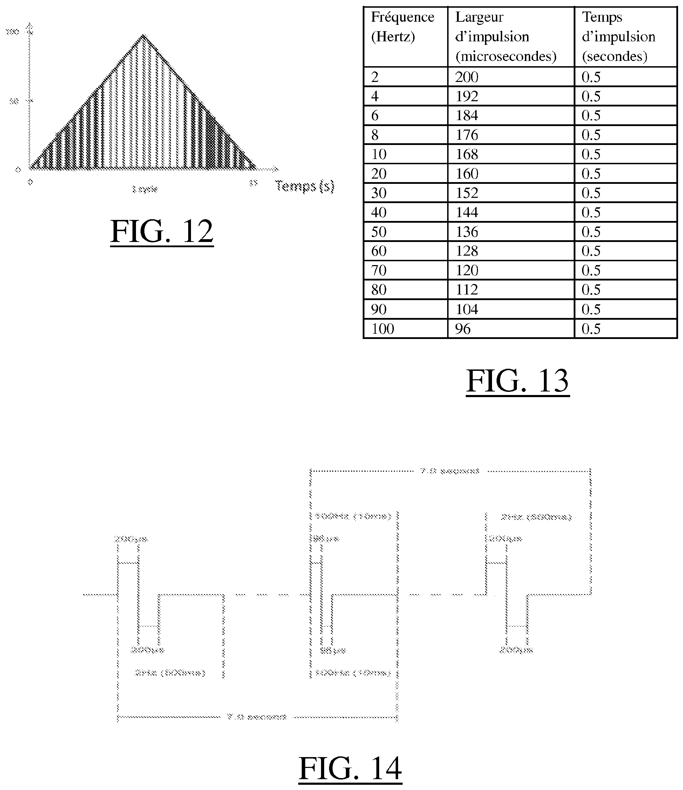

Dans des exemples, la mémoire du dispositif peut stocker un quatrième programme schématiquement représenté sur la FIG. 12. Il s'agit de générer des successions de cycles d'impulsions de courant où pour chaque cycle la fréquence varie en fonction du temps afin de former un signal triangle : la fréquence croit graduellement pendant la première moitié du cycle et décroit graduellement pendant la deuxième moitié du cycle pour atteindre en fin de cycle une fréquence qui est égale à la fréquence de départ de la première moitié du cycle. Dans des exemples, la fréquence des impulsions du courant électrique est comprise entre 1 et 150 hertz bornes incluses. La largueur des impulsions diminue à chaque augmentation de la fréquence des impulsions, et inversement augmente à chaque diminution de la fréquence des impulsions. En d'autres termes, la première moitié du cycle voit la largeur des impulsions graduellement diminuer tandis que la deuxième moitié du cycle voit la largeur des impulsions graduellement augmenter. La valeur de tension du courant électrique peut être comprise entre 3,5 et 21 volts bornes incluses. La FIG. 13 est un exemple de variations de la fréquence et de la largeur des impulsions pour un demi-cycle. Les mêmes valeurs inversées peuvent être utilisées pour le demi-cycle suivant. La largeur des impulsions du courant électrique peut être comprise entre 50 et 500 microsecondes bornes incluses, de préférence entre 50 et 250 microsecondes bornes incluses. Dans l'exemple de la FIG. 13, le temps d'impulsion de toutes les impulsions est égal à 0.5 secondes, et la somme des temps d'impulsion pour un demi-cycle est de 7 secondes. Un cycle complet est donc de 14 secondes. On note que sur la FIG. 14 que l'intensité des impulsions est représentée en fonction du temps et que les impulsions sont bidirectionnelles. Les valeurs reportées sur la FIG. 14 correspondent à celles du tableau de la FIG. 13. On peut ainsi observer sur la FIG. 14 que la première impulsion comprend deux largeurs d'impulsion de 200 microsecondes sur un temps d'impulsion de 0.5 secondes à une fréquence de 2 Hertz, et la dernière impulsion du demi-cycle (montant) comprend deux impulsions de 96 microsecondes sur un temps d'impulsion de 0.5 secondes et à une fréquence de 100 Hertz. Cette dernière impulsion du demi-cycle montant est également la première impulsion du demi-cycle descendant, et la première impulsion du demi-cycle montant devient la dernière impulsion du demi-cycle descendant. Dans des exemples les cycles successifs peuvent être générés continuellement, c'est-à-dire sans pause entre eux. Dans des exemples, et de préférence, les cycles sont discontinus avec une pause entre chaque cycle pouvant être comprise par exemple entre 1 et 3 secondes. Le quatrième programme provoque un soulagement rapide et durable de la douleur accompagné d'une sensation de massage qui est notamment causée par la variation progressive et simultanée de la fréquence et de la largeur d'impulsion du signal électrique.

Dans des exemples, la mémoire du dispositif peut stocker un cinquième programme qui exécute successivement les premier, troisième et quatrième programmes de manière répétée. Entre chaque répétition, aucun signal électrique n'est émis afin que chaque exécution successive des trois programmes dure entre 45 et 75 secondes, de préférence autour de 60 secondes.

Dans des exemples, la mémoire du dispositif peut stocker un sixième programme qui est une combinaison du premier programme exécuté successivement avec une fréquence respective sensiblement égale à 100 hertz, puis 80 hertz et puis 60 hertz, et du deuxième programme exécuté successivement avec une fréquence respective sensiblement égale à 100/2 hertz, puis 80/2 hertz et puis 60/2 hertz. La largeur des impulsions peut être comprise entre 80 et 120 microsecondes, et de préférence sensiblement égale à 100 microsecondes. Ce programme est particulièrement bien adapté pour les dysménorrhées.

Dans des exemples, la mémoire du dispositif peut stocker un septième programme semblable au premier programme, pour lequel la fréquence des impulsions est comprise entre 80 et 120 hertz, de préférence sensiblement égale 100 hertz et la largeur des impulsions est comprise entre 80 et 120 microsecondes, de préférences sensiblement égale 100 microsecondes. Les dysménorrhées peuvent être traitées à l'aide de ce septième programme. Les douleurs liées aux maux de têtes peuvent également être efficacement diminuées avec ce programme.

Dans des exemples, la mémoire du dispositif peut stocker un huitième programme semblable au premier programme pour lequel la fréquence des impulsions est comprise entre 40 et 80 hertz, de préférence sensiblement égale 60 hertz et la largeur des impulsions est comprises entre 80 et 120 microsecondes, de préférences sensiblement égale 100 microsecondes. Ce huitième programme est particulièrement adapté pour prévenir les maux de tête, par exemple une migraine, lorsque les premiers signes apparaissent.

Dans des exemples, la durée des programmes susmentionnés peut être comprise entre 20 et 35 minutes, de préférence la durée d'un programme est sensiblement égale à 30 minutes, voire 60 minutes pour une meilleure efficacité tout en préservant le niveau de charge de la batterie.

Dans des exemples, la valeur de la tension du courant électrique pour les programmes est comprise entre 3,5 et 15 volts, bornes incluses. Ces valeurs ont montré une bonne efficacité de la stimulation tout en étant adaptées aux tensions que peut délivrer la batterie rechargeable.

De retour sur la FIG. 16, des exemples de sélection d'un programme stocké sur la mémoire sont discutés. Sur la FIG. 16, la sélection est présentée comme étant réalisée après la mise sous tension du dispositif, on comprendra cependant que la sélection peut être réalisée à n'importe quel moment, par exemple il est possible de sélectionner un autre programme alors même qu'un programme est en cours d'exécution. De manière générale, une condition nécessaire et suffisante pour qu'un programme puisse être sélectionné est que le dispositif soit sous tension.

Dans des exemples, la sélection comprend la sélection d'un programme par défaut. Par exemple, si la mémoire comprend plusieurs programmes, l'un de ces programme est automatiquement sélectionné pour générer le courant électrique délivré par l'électrode. Dans un exemple, ce programme par défaut est le premier programme précédemment discuté qui permet de soulager rapidement une douleur. La sélection du programme par défaut peut être accompagnée par la fourniture d'une valeur par défaut pour chaque paramètre du programme par défaut ; chaque valeur est comprise dans la mémoire.

Dans des exemples, l'utilisateur peut sélectionner un programme (1320), ou bien encore changer le programme par défaut si celui-ci a été sélectionné suite à la mise sous tension du dispositif. Sur la FIG. 16, la sélection est effectuée suite à la sélection par défaut du premier programme (opération non représentée). La sélection est obtenue par une action utilisateur sur un organe de sélection (1310), par exemple un bouton disposé sur le dispositif, qui déclenche la sélection d'un autre programme compris dans la mémoire. Lorsque la mémoire comprend plusieurs programmes, les programmes peuvent être sélectionnés séquentiellement, c'est-à-dire que chaque nouvelle action utilisateur sur l'organe de sélection déclenche la sélection d'un nouveau programme, et lorsque tous les programmes ont été proposés à l'utilisateur, le programme par défaut est à nouveau proposé pour une nouvelle action utilisateur sur l'organe de sélection. Cela permet à l'utilisateur de faire défiler continuellement tous les programmes un par un. Le dernier programme sélectionné est le dernier programme présenté à l'utilisateur, soit le programme sélectionné par défaut soit le programme sélectionné suite à la dernière action utilisateur sur l'organe de sélection.

Dans des exemples, l'organe de sélection permet de proposer un nouveau programme, mais également de proposer un programme précédemment présenté. Cela permet à l'utilisateur de sélectionner plus rapidement le programme qu'il souhaite avoir par exemple au cas où il l'aurait manqué. Dans un exemple, l'organe de sélection comprend deux organes de sélection, par exemple un bouton « plus » et un bouton « moins », où chaque organe permet de faire défiler les programmes dans un sens qui lui est propre ; par exemple le bouton « plus » permet de progresser dans une liste ordonnée des programmes dans un sens (par exemple dans le sens croissant) alors que le bouton « moins » permet de progresser dans la liste dans un sens inverse.

Dans des exemples, la sélection d'un programme peut être accompagnée d'un son qui identifie de manière unique le programme sélectionné. On comprend que le dispositif comprend alors un dispositif d'émission de son, qui peut être mais n'est pas limité à, un hautparleur, un buzzer,... L'identification du programme sélectionné peut être réalisée par exemple à l'aide d'un nombre de bip où chaque nombre est associé à un seul programme ; par exemple le premier programme avec un bip, le deuxième avec deux bips, et ainsi de suite.

Dans des exemples, la sélection peut être accompagnée d'un signal lumineux émis par le dispositif d'émission de lumière.

Dans des exemples, la sélection peut être accompagnée (1322) d'un son qui identifie de manière unique le programme sélectionné, comme précédemment sélectionné, ou encore d'un son qui a pour unique but d'informer que la sélection est confirmée.

Ensuite, une fois la sélection opérée, l'unité de commande est configurée pour que le courant électrique délivré par l'électrode corresponde aux valeurs des paramètres du programme sélectionné. En d'autres termes, l'unité de commande est configurée pour exécuter la stimulation électrique associée au programme. Cette configuration est donc une programmation (ou une reprogrammation) du dispositif.

Dans des exemples, la sélection peut être suivie d'une confirmation de la sélection. La confirmation de la sélection permet à l'utilisateur de confirmer au dispositif qu'il souhaite que le programme sélectionné soit exécuté. Dans un exemple, cela peut être réalisé avec une action utilisateur particulière sur l'organe de sélection.

Dans des exemples, la confirmation de la sélection peut être implicite avec le démarrage de la stimulation (et donc une fois que l'unité de commande a été configurée), par exemple en effectuant une action utilisateur sur l'organe de sélection (1330).

Dans des exemples, la sélection du programme peut comprendre une action utilisateur permettant de sélectionner (1340) une nouvelle valeur d'un paramètre du programme sélectionné. La sélection de cette valeur de paramètre peut être faite via un organe de sélection.

Dans des exemples, l'organe servant à sélectionner cette valeur de paramètre peut être le même que celui utilisé pour sélectionner le programme. Dans un exemple, l'organe de sélection comprend deux boutons, un bouton « plus » et un bouton « moins ». Chaque action sur le bouton « plus » augmente la valeur, et inversement chaque action utilisateur sur le bouton « moins » diminue la valeur du paramètre (1342). Afin de pouvoir faire la distinction entre une sélection de programme et une modification d'une valeur de paramètre lorsque le même organe de sélection est utilisé, la durée de l'action utilisateur sur l'organe peut être utilisée : une action longue (par exemple appuyer plus de trois secondes sur le bouton) sera interprétée par le dispositif comme étant une sélection de programme, et inversement une action courte (par exemple appuyer moins de trois secondes sur le bouton) sera interprétée par le dispositif comme étant une sélection de valeur de paramètre.

Dans des exemples, le choix d'une valeur de paramètre peut être accompagné d'un son et/ou d'un signal lumineux pour assister l'utilisateur dans la sélection.

Par exemple, la plage de valeurs de paramètre pouvant être sélectionnée peut-être limitée. Lorsque l'utilisateur souhaite sortir de cette plage de valeurs, ce qui n'est pas possible, le système peut en informer l'utilisateur à l'aide d'un son et/ou d'un signal lumineux.

Dans des exemples, la nouvelle valeur sélectionnée est une valeur d'intensité du courant électrique. Cela permet à l'utilisateur d'augmenter l'effet de l'électrostimulation en fonction de son ressenti de la stimulation électrique et du soulagement de sa douleur obtenu.

On comprend que le choix de la valeur de l'intensité pourrait être réalisé avant le commencement de la stimulation, par exemple, l'utilisateur sait quelle intensité lui convient.

Dans des exemples, lorsque la batterie n'est pas suffisamment chargée pour réaliser un programme complet, le dispositif peut en informer l'utilisateur et/ou se mettre en sécurité (le programme ne peut pas être exécuté) ou alors être exécuté jusqu'à ce que la batterie soit vide.

Dans des exemples, lorsque la batterie n'est pas suffisamment chargée pour réaliser un programme complet (dans toute sa durée), il est possible de faire fonctionner le dispositif en reliant la batterie rechargeable à une source d'énergie externe. Le dispositif peut alors fonctionner sur secteur.

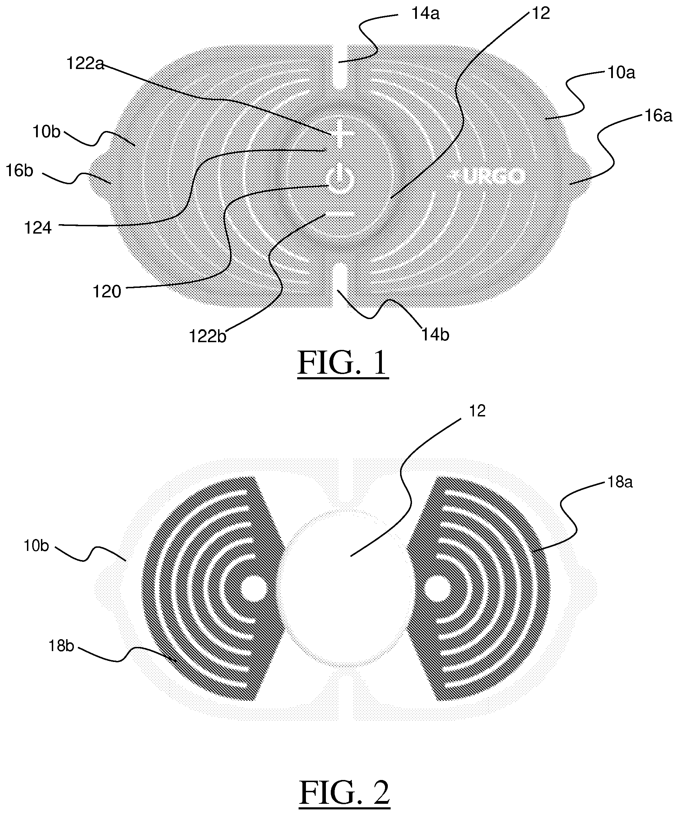

En référence aux FIG. 1 à FIG. 6, des exemples de dispositifs sont maintenant discutés. Ces figures sont des photographies de dispositifs. Sur la FIG. 1, le dispositif selon l'invention comprend un boîtier dans lequel la batterie, l'unité de commande et la mémoire sont agencées. Le boîtier permet de les protéger d'éléments externes pouvant les détériorer (poussière, eau...). Le boîtier peut donc être étanche. Le boîtier peut comprendre un organe de mise sous tension et/ou de mise hors tension 120. Le boîtier peut également comprendre un premier organe de sélection d'un programme et/ou un deuxième organe de sélection d'une valeur de paramètre. Dans l'exemple de la FIG. 1, le premier et le deuxième organe sont confondus et identiques : il s'agit d'un bouton « plus » 122a et d'un bouton « moins » 122b. Le boîtier de cet exemple comprend une LED 124 agissant comme indicateur lumineux et un buzzer (non représenté). Le boîtier comprend deux électrodes, une positive et une négative, qui ne sont pas directement visibles avec la vue de dessus ; plus précisément, la deuxième face (isolante) de l'électrode 10a, 10b est visible. Les deux électrodes sont reliées et solidaires au boîtier, formant ainsi un dispositif compact. Deux encoches 14a et 14b augmentent la flexibilité du dispositif, permettant ainsi de le placer sur des parties du corps courbes.

Le boîtier peut être dans une matière polymère solide et protectrice, par exemple du plastique ABS (Acrylonitrile butadiene styrene). Le boîtier 12 et les électrodes 10a, 10b peuvent être recouverts sur cette face d'une matière souple et électriquement isolante de type silicone. Les électrodes comprennent en outre dans cet exemple des languettes 16a, 16b qui facilitent le retrait du dispositif qui est maintenu sur la peau par exemple à l'aide d'un gel adhésif.

La FIG. 2 est une vue de dessous du même dispositif représenté sur la FIG. 1. Le boîtier 12 comprend un fond qui forme une surface, par exemple un plan. Cette surface est de préférence confondue avec la surface des électrodes 10a, 10b afin d'améliorer le placement du dispositif sur la zone à traiter. L'électrode est donc sensiblement dans le même plan que le fond du boîtier. Les surfaces conductrices 16a, 16b d'électricité de l'électrode sont accessibles. Ces surfaces peuvent accueillir le gel, par exemple le gel adhésif.

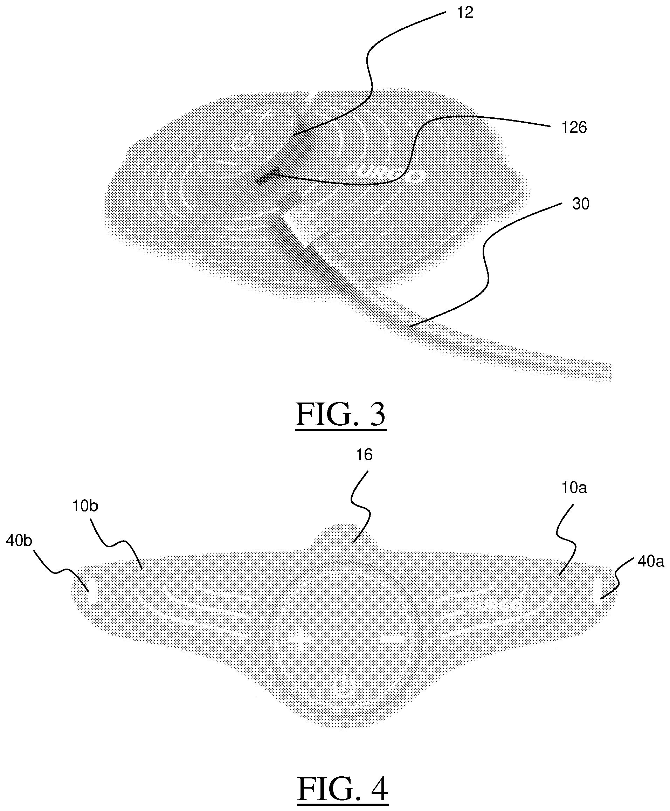

La FIG. 3 est une vue de côté du dispositif d'électrostimulation transcutanée représenté sur les FIG. 1 et FIG. 2. Le boîtier 12 comprend une ouverture 126 qui donne accès à une interface permettant de relier électriquement la batterie à une source d'électricité externe, par exemple pour recharger la batterie. L'interface peut être de tout type. Dans un exemple, l'interface est une interface USB et elle peut être reliée à une source électrique à l'aide d'un câble USB 30.

Le dispositif représenté sur les FIGs. 1 à 3 comprend de larges électrodes qui permettent de recouvrir des parties du corps relativement planes, par exemple le dos ou une cuisse.

La FIG. 4 est un autre exemple de dispositif semblable à celui des FIGs. 1 à 3, excepté que les électrodes 10a, 10b ont une forme sensiblement différente qui est adaptée pour être disposée sur le front afin de soulager les maux de tête. Ainsi les électrodes sont moins larges et plus longues pour couvrir le front. Une seule languette est présente dans cet exemple. Les électrodes comprennent chacune une ouverture 40a, 40b facilitant l'accroche d'un bandeau (non représenté) afin de maintenir le dispositif contre le front du patient.

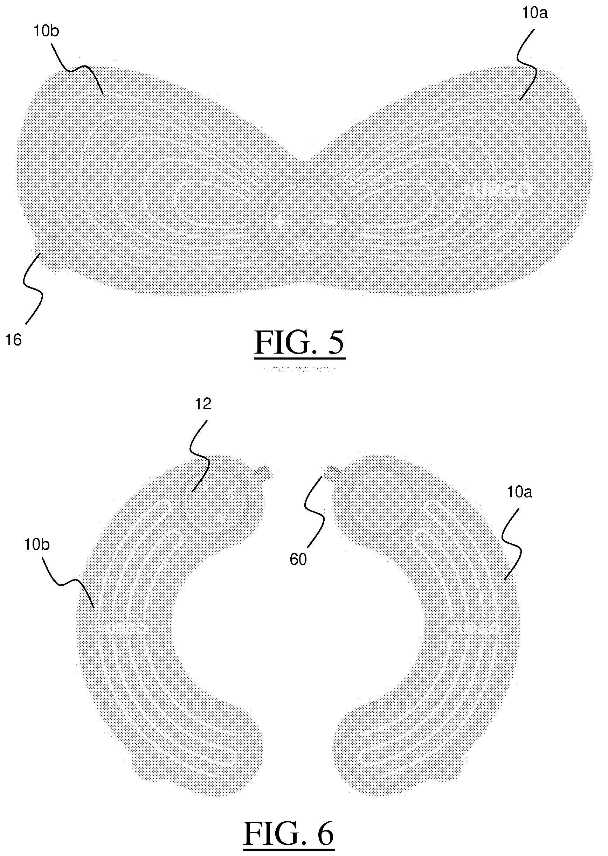

La FIG. 5 est un autre exemple de dispositif semblable à celui des FIGs. 1 à 3, excepté que les électrodes 10a, 10b ont une forme sensiblement différente qui est adaptée pour être disposée sur le ventre afin de soulager les dysménorrhées. Notamment leur forme est adaptée pour recouvrir les organes responsables des douleurs lorsque le dispositif est placé sur le ventre.

La FIG. 6 est un autre exemple de dispositif semblable à celui des FIGs. 1 à 3, excepté que l'électrode positive 16a (ou inversement négative 16b) n'est plus solidaire du boîtier. Le dispositif comprend deux éléments, un premier élément comprenant le boîtier et l'électrode positive (ou inversement négative), et un deuxième élément comprenant l'électrode négative (ou inversement négative). Le deuxième élément 10a est relié au boîtier via un fil conducteur d'électricité, par exemple le câble 60. Les deux éléments ont sensiblement une forme de demi-disque qui est particulièrement adaptée pour être déposée autour d'une articulation, par exemple un genou. Cela améliore l'efficacité de l'électrostimulation.

Dans des exemples, le boîtier peut en outre comprendre des moyens de communication sans fil permettant de configurer l'unité de commande, par exemple réaliser l'opération de sélection d'un programme et/ou l'opération de sélection d'une valeur de paramètre. La configuration peut par exemple s'effectuer avec le protocole Bluetooth^{©} entre une application par exemple exécuté par un smartphone et l'unité de commande.

## Revendications

1. Procédé de réglage d'un dispositif (600) d'électrostimulation
transcutanée, le dispositif comprenant :
- au moins une électrode (610) ;
- une batterie (606) rechargeable connectée à l'électrode (610) ;
- une mémoire (604) sur laquelle sont stockés plusieurs programmes, les programmes stockés incluant un programme par défaut ;
- une unité de commande (608) couplée à la mémoire (604), l'unité de commande (608) étant apte à configurer un courant électrique fourni par la batterie (606) et délivré par l'électrode (610) ;
le procédé comprenant les opérations consistant à :
- sélectionner (1320), dans la mémoire (604), un programme associé à plusieurs paramètres du courant électrique délivré par l'électrode (610), chaque paramètre étant associé à une valeur, le programme comprenant au moins comme paramètres :
- une tension des impulsions du courant électrique ;
- une durée des impulsions du courant électrique ;
- une fréquence des impulsions du courant électrique ;
- configurer l'unité de commande (608) pour que le courant électrique délivré par l'électrode (610) corresponde aux valeurs des paramètres du programme sélectionné,
dans lequel sélectionner un programme comprend les opérations consistant à :
- sélectionner le programme par défaut lors d'une mise sous tension du dispositif ;
- fournir une valeur par défaut, stockée dans la mémoire (604),
associée à chaque paramètre du programme par défaut sélectionné.

2. Procédé selon la revendication 1, dans lequel sélectionner un programme comprend en outre l'opération consistant à :
- sélectionner, par action utilisateur sur un premier organe de sélection, une nouvelle valeur d'au moins un paramètre du courant électrique délivré par l'électrode.

3. Procédé selon la revendication 2, dans lequel la nouvelle valeur sélectionnée du au moins un paramètre est une valeur d'intensité d'un courant électrique.

4. Procédé selon l'une quelconque des revendications précédentes, dans lequel sélectionner un programme comprend en outre l'opération consistant à :
- sélectionner séquentiellement, par action utilisateur sur un deuxième organe de sélection, un programme parmi au moins deux programmes stockés sur la mémoire.

5. Procédé selon l'une quelconque des revendications précédentes, dans lequel une opération de sélection comprend en outre :
- émettre, par un dispositif d'émission de lumière, un signal lumineux pour chaque nouvelle sélection.

6. Procédé selon l'une quelconque des revendications précédentes, dans lequel une des opérations consistant à sélectionner un programme comprend en outre :
- émettre, par un dispositif d'émission de son, un son qui identifie de manière unique le programme sélectionné.

7. Procédé selon l'une quelconque des revendications précédentes, la mémoire stockant un premier programme pour lequel :
- la largeur des impulsions du courant électrique délivré par l'électrode est comprise en 50 et 500 microsecondes bornes incluses, de préférence entre 150 et 250 microsecondes bornes incluses ;
- la fréquence des impulsions du courant électrique délivré par l'électrode est comprise entre 60 et 140 hertz bornes incluses, de préférence entre 80 et 120 hertz bornes incluses.

8. Procédé selon l'une quelconque des revendications précédentes, la mémoire stockant un deuxième programme pour lequel :
- la largeur des impulsions du courant électrique est comprise en 50 et 500 microsecondes bornes incluses, de préférence entre 150 et 250 microsecondes bornes incluses ;
- la fréquence des impulsions du courant électrique est comprise entre 2 et 8 hertz bornes incluses, de préférence entre 4 et 6 hertz bornes incluses.

9. Procédé selon l'une quelconque des revendications 7 ou 8, dans lequel les impulsions du courant électrique délivré par l'électrode sont générées en continu.

10. Procédé selon l'une quelconque des revendications précédentes, la mémoire stockant un troisième programme pour lequel :
- la largeur des impulsions du courant électrique est comprise en 50 et 500 microsecondes bornes incluses, de préférence entre 150 et 250 microsecondes bornes incluses ;
- la fréquence des impulsions du courant électrique délivré par l'électrode comprend :
- - une première fréquence d'impulsion du courant électrique comprise entre 60 et 140 hertz bornes incluses, de préférence entre 80 et 120 hertz bornes incluses ; et
- - une deuxième fréquence d'impulsion du courant électrique comprise entre 2 et 8 hertz bornes incluses, de préférence entre 4 et 6 hertz bornes incluses ;
- - une première durée de génération des impulsions selon la première fréquence et une deuxième durée de génération des impulsions selon la deuxième fréquence, la première durée et la deuxième durée étant sensiblement égales.

11. Procédé selon l'une quelconque des revendications précédentes, la mémoire stockant un quatrième programme pour lequel :
- la fréquence des impulsions du courant électrique délivré par l'électrode est comprise entre 1 et 150 hertz bornes incluses, la fréquence des impulsions augmentant et diminuant en fonction du temps afin de former un signal triangle ;
- la largeur des impulsions du courant électrique délivré par l'électrode est comprise en 50 et 500 microsecondes bornes incluses, de préférence entre 50 et 250 microsecondes bornes incluses, la largueur de l'impulsion diminuant pour chaque augmentation de la fréquence des impulsions et augmentant pour chaque diminution de la fréquence des impulsions.

12. Procédé selon l'une quelconque des revendications 10 ou 11, dans lequel les impulsions du courant électrique délivré par l'électrode sont générées par cycles discontinus.

13. Procédé selon la combinaison des revendications 7 à 12, dans lequel la mémoire stocke un cinquième programme qui exécute successivement les premier, deuxième, troisième et quatrième programmes de manière répétée, aucun signal électrique n'étant émis entre chaque répétition afin que chaque exécution successive des trois programmes ait une durée comprise entre 45 et 75 secondes, de préférence sensiblement égale à 60 secondes.

14. Procédé selon l'une quelconque des revendications 7 à 13, dans lequel la valeur de tension du courant électrique est comprise entre 3,5 et 25 volts, bornes incluses.

15. Programme d'ordinateur comprenant des instructions de code de programme pour l'exécution des étapes du procédé selon l'une quelconque des revendications 1 à 14 lorsque ledit programme est exécuté sur une unité de commande du dispositif d'électrostimulation transcutanée.

16. Dispositif d'électrostimulation transcutanée (600), comprenant :
- au moins une électrode (610) ;
- une batterie (606) rechargeable connectée à l'électrode ;
- une mémoire (604) sur laquelle sont stockés plusieurs programmes, les programmes stockés incluant un programme par défaut ;
- une unité de commande (608) couplée à la mémoire (604), l'unité de commande (608) étant adaptée pour configurer un courant électrique fourni par la batterie (606) et délivré par l'électrode (610), la mémoire (604) comprenant des instructions de code de programme du programme selon la revendication 15.

17. Dispositif selon la revendication 16, dans lequel :
- la batterie (606), l'unité de commande (608) et la mémoire (604) sont agencées dans un boîtier comprenant un fond formant une surface ;
- l'électrode (610) est disposée sur un matériau souple qui est sensiblement dans le même plan que le fond du boîtier.

## Patentansprüche

1. Verfahren zum Einstellen einer Vorrichtung (600) zur transkutanen Elektrostimulation, die Vorrichtung umfassend:
- mindestens eine Elektrode (610);
- eine wiederaufladbare Batterie (606), die mit der Elektrode (610) verbunden ist;
- einen Speicher (604), in dem mehrere Programme gespeichert sind, wobei die gespeicherten Programme ein Standardprogramm beinhalten;
- eine Steuereinheit (608), die mit dem Speicher (604) gekoppelt ist, wobei die Steuereinheit (608) geeignet ist, um einen elektrischen Strom zu konfigurieren, der von der Batterie (606) bereitgestellt und von der Elektrode (610) zugeführt wird;
das Verfahren umfassend die Vorgänge, die aus Folgendem bestehen:
- Auswählen (1320), in dem Speicher (604), eines Programms, das mit mehreren Parametern des von der Elektrode (610) zugeführten elektrischen Stroms assoziiert ist, wobei mit jedem Parameter ein Wert assoziiert ist, das Programm umfassend als Parameter zumindest:
- eine Spannung von Impulsen des elektrischen Stroms;
- eine Dauer von Impulsen des elektrischen Stroms;
- eine Frequenz von Impulsen des elektrischen Stroms;
- Konfigurieren der Steuereinheit (608), sodass der von der Elektrode (610) zugeführte elektrische Strom den Werten der Parameter des ausgewählten Programms entspricht,
wobei ein Auswählen eines Programms die Vorgänge umfasst, die aus Folgendem bestehen:
- Auswählen des Standardprogramms, wenn die Vorrichtung eingeschaltet wird;
- Bereitstellen eines Standardwerts, der in dem Speicher (604) gespeichert ist und mit jedem Parameter des ausgewählten Standardprogramms assoziiert ist.

2. Verfahren nach Anspruch 1, wobei ein Auswählen eines Programms ferner den Vorgang umfasst, der aus Folgendem besteht:
- Auswählen, durch Benutzereinwirkung auf ein erstes Auswahlorgan, eines neuen Werts für mindestens einen Parameter des von der Elektrode zugeführten elektrischen Stroms.

3. Verfahren nach Anspruch 2, wobei der neu ausgewählte Wert des mindestens einen Parameters ein Wert für die Stärke eines elektrischen Stroms ist.

4. Verfahren nach einem der vorherigen Ansprüche, wobei ein Auswählen eines Programms ferner den Vorgang umfasst, der aus Folgendem besteht:
- nacheinander Auswählen, durch Benutzereinwirkung auf ein zweites Auswahlorgan, eines Programms aus mindestens zwei in dem Speicher gespeicherten Programmen.

5. Verfahren nach einem der vorherigen Ansprüche, wobei ein Auswahlvorgang ferner Folgendes umfasst:
- Emittieren, durch eine Lichtemissionsvorrichtung, eines Lichtsignals für jede neue Auswahl.

6. Verfahren nach einem der vorherigen Ansprüche, wobei einer der Vorgänge, die aus einem Auswählen eines Programms bestehen, ferner Folgendes umfasst:
- Emittieren, durch eine Tonemissionsvorrichtung, eines Tons, der das ausgewählte Programm eindeutig identifiziert.

7. Verfahren nach einem der vorherigen Ansprüche, wobei der Speicher ein erstes Programm speichert, für das:
- die Impulsbreite des von der Elektrode zugeführten elektrischen Stroms zwischen 50 und 500 Mikrosekunden einschließlich, vorzugsweise zwischen 150 und 250 Mikrosekunden einschließlich, liegt;
- die Impulsfrequenz des von der Elektrode zugeführten elektrischen Stroms zwischen 60 und 140 Hertz einschließlich, vorzugsweise zwischen 80 und 120 Hertz einschließlich, liegt.

8. Verfahren nach einem der vorherigen Ansprüche, wobei der Speicher ein zweites Programm speichert, für das:
- die Impulsbreite des elektrischen Stroms zwischen 50 und 500 Mikrosekunden einschließlich, vorzugsweise zwischen 150 und 250 Mikrosekunden einschließlich, liegt;
- die Frequenz der Impulse des elektrischen Stroms zwischen 2 und 8 Hertz einschließlich, vorzugsweise zwischen 4 und 6 Hertz einschließlich, liegt.

9. Verfahren nach einem der Ansprüche 7 oder 8, wobei die Impulse des von der Elektrode zugeführten elektrischen Stroms kontinuierlich erzeugt werden.

10. Verfahren nach einem der vorherigen Ansprüche, wobei der Speicher ein drittes Programm speichert, für das:
- die Impulsbreite des elektrischen Stroms zwischen 50 und 500 Mikrosekunden einschließlich, vorzugsweise zwischen 150 und 250 Mikrosekunden einschließlich, liegt;
- die Frequenz der Impulse des von der Elektrode zugeführten elektrischen Stroms Folgendes umfasst:
- - eine erste Impulsfrequenz des elektrischen Stroms, die zwischen 60 und 140 Hertz einschließlich, vorzugsweise zwischen 80 und 120 Hertz einschließlich, liegt; und
- - eine zweite Impulsfrequenz des elektrischen Stroms, die zwischen 2 und 8 Hertz einschließlich, vorzugsweise zwischen 4 und 6 Hertz einschließlich liegt;
- - eine erste Erzeugungsdauer von Impulsen gemäß der ersten Frequenz und eine zweite Erzeugungsdauer von Impulsen gemäß der zweiten Frequenz, wobei die erste Dauer und die zweite Dauer im Wesentlichen gleich sind.

11. Verfahren nach einem der vorherigen Ansprüche, wobei der Speicher ein viertes Programm speichert, für das:
- die Impulsfrequenz des von der Elektrode zugeführten elektrischen Stroms zwischen 1 und 150 Hertz einschließlich liegt, wobei die Impulsfrequenz mit der Zeit ansteigt und abnimmt, um ein Dreieckssignal zu bilden;
- die Impulsbreite des von der Elektrode zugeführten elektrischen Stroms zwischen 50 und 500 Mikrosekunden einschließlich, vorzugsweise zwischen 50 und 250 Mikrosekunden einschließlich liegt, wobei die Impulsbreite mit jedem Anstieg der Impulsfrequenz abnimmt und mit jedem Abfall der Impulsfrequenz zunimmt.

12. Verfahren nach einem der Ansprüche 10 oder 11, wobei die Impulse des von der Elektrode zugeführten elektrischen Stroms in diskontinuierlichen Zyklen erzeugt werden.

13. Verfahren nach der Kombination der Ansprüche 7 bis 12, wobei der Speicher ein fünftes Programm speichert, das nacheinander das erste, das zweite, das dritte und das vierte Programm wiederholt ausführt, wobei zwischen jeder Wiederholung kein elektrisches Signal emittiert wird, sodass jede aufeinanderfolgende Ausführung der drei Programme eine Dauer zwischen 45 und 75 Sekunden aufweist, vorzugsweise im Wesentlichen gleich 60 Sekunden ist.

14. Verfahren nach einem der Ansprüche 7 bis 13, wobei der Spannungswert des elektrischen Stroms zwischen 3,5 und 25 Volt einschließlich liegt.

15. Computerprogramm, umfassend Programmcodeanweisungen zur Ausführung der Schritte des Verfahrens nach einem der Ansprüche 1 bis 14, wenn das Programm auf einer Steuereinheit der Vorrichtung zur transkutanen Elektrostimulation ausgeführt wird.

16. Vorrichtung zur transkutanen Elektrostimulation (600), umfassend:
- mindestens eine Elektrode (610);
- eine wiederaufladbare Batterie (606), die mit der Elektrode verbunden ist;
- einen Speicher (604), in dem mehrere Programme gespeichert sind, wobei die gespeicherten Programme ein Standardprogramm beinhalten;
- eine Steuereinheit (608), die mit dem Speicher (604) gekoppelt ist, wobei die Steuereinheit (608) angepasst ist, um einen elektrischen Strom zu konfigurieren, der von der Batterie (606) bereitgestellt und von der Elektrode (610) zugeführt wird, der Speicher (604) umfassend Programmcodeanweisungen des Programms nach Anspruch 15.

17. Vorrichtung nach Anspruch 16, wobei:
- die Batterie (606), die Steuereinheit (608) und der Speicher (604) in einem Gehäuse angeordnet sind, das einen Boden aufweist, der eine Oberfläche bildet;
- die Elektrode (610) auf einem flexiblen Material angeordnet ist, das im Wesentlichen in der gleichen Ebene wie der Boden des Gehäuses ist.

## Claims

1. A method of adjusting a transcutaneous electrical stimulation device (600), the device comprising:
- at least one electrode (610),
- a rechargeable battery (606) connected to the electrode (610),
- a memory (604) on which a plurality of programs are stored, the stored programs including a default program,
- a control unit (608) coupled to the memory (604), the control unit (608) being able to configure an electric current supplied by the battery (606) and delivered by the electrode (610),
the method comprising the steps consisting of:
- selecting (1320), in the memory (604), a program associated with a plurality of parameters of the electric current delivered by the electrode (610), each parameter being associated with a value, the program comprising at least as parameters:
- a voltage of the pulses of the electric current,
- a duration of the pulses of the electric current,
- a frequency of the pulses of the electric current,
- configuring the control unit (608) so that the electric current delivered by the electrode (610) corresponds to the parameter values of the selected program,
wherein selecting a program comprises the operations consisting of:
- selecting the default program when powering up the device:
- providing a default value, stored in the memory (604), associated with each parameter of the selected default program.

2. The method according to claim 1, wherein selecting a program further comprises the operation consisting of:
- selecting, by an action performed by a user on a first selector member, a new value of at least one parameter of the electric current delivered by the electrode.

3. The method according to claim 2, wherein the new selected value of the at least one parameter is a value of the intensity of an electric current.

4. The method according to any of the preceding claims, wherein selecting a program further comprises the operation consisting of:
- sequentially selecting, by an action performed by a user on a second selector member, a program amongst at least two programs stored in the memory.

5. The method according to any of the preceding claims, wherein a selection operation further comprises:
- emitting, by means of a light emitting device, a light signal for each new selection.

6. The method according to any of the preceding claims, wherein one of the operations of selecting a program further comprises:
- emitting, by means of a sound emitting device, a sound that uniquely identifies the selected program.

7. The method according to any of the preceding claims, wherein the memory stores a first program for which:
- the width of the pulses of the electric current delivered by the electrode is comprised between 50 and 500 microseconds inclusive, preferably between 150 and 250 microseconds inclusive,
- the frequency of the pulses of the electric current delivered by the electrode is comprised between 60 and 140 hertz inclusive, preferably between 80 and 120 hertz inclusive.

8. The method according to any of the preceding claims, the memory storing a second program for which:
- the width of the pulses of the electric current is comprised between 50 and 500 microseconds inclusive, preferably between 150 and 250 microseconds inclusive,
- the frequency of the pulses of the electric current is comprised between 2 and 8 hertz inclusive, preferably between 4 and 6 hertz inclusive.

9. The method according to any of claims 7 or 8, wherein the pulses of the electric current delivered by the electrode are generated continuously.

10. The method according to any of the preceding claims, wherein the memory stores a third program for which:
- the width of the pulses of the electric current is comprised between 50 and 500 microseconds inclusive, preferably between 150 and 250 microseconds inclusive,
- the frequency of the pulses of the electric current delivered by the electrode comprises:
- a first frequency of a pulse of the electric current comprised between 60 and 140 hertz inclusive, preferably between 80 and 120 hertz inclusive, and
- a second frequency of a pulse of the electric current comprised between 2 and 8 hertz inclusive, preferably between 4 and 6 hertz inclusive,
- a first duration of generation of pulses at the first frequency and a second duration of generation of pulses at the second frequency, the first duration and the second duration being substantially equal.

11. The method according to any of the preceding claims, the memory storing a fourth program for which:
- the frequency of the pulses of the electric current delivered by the electrode is comprised between 1 and 150 hertz inclusive, the frequency of the pulses increasing and decreasing as a function of time in order to form a triangular signal,
- the width of the pulses of the electric current delivered by the electrode is comprised between 50 and 500 microseconds inclusive, preferably between 50 and 250 microseconds inclusive, the width of the pulse decreasing for each increase in the frequency of the pulses and increasing for each decrease in the frequency of the pulses.

12. The method according to any of claims 10 or 11, wherein the pulses of the electric current delivered by the electrode are generated in discontinuous cycles.

13. The method according to the combination of claims 7 to 12, wherein the memory stores a fifth program which successively executes the first, second, third and fourth programs repeatedly, no electrical signal being emitted between each repetition so that each successive execution of the three programs has a duration comprised between 45 and 75 seconds, preferably substantially equal to 60 seconds.

14. The method according to any of claims 7 to 13, wherein the voltage value of the electric current is between comprised 3.5 and 25 volts, inclusive.

15. A computer program product comprising program code instructions for executing the steps of the method according to any of claims 1 to 14, wherein said program is executed on a control unit of the transcutaneous electrical stimulation device.

16. A transcutaneous electrical stimulation device (600), comprising:
- at least one electrode (610),
- a rechargeable battery (606) connected to the electrode,
- a memory (604) on which a plurality of programs is stored, the stored programs including a default program,
- a control unit (608) coupled to the memory (604), the control unit (608) being suitable for configuring an electrical current supplied by the battery (606) and delivered by the electrode (610), the memory (604) comprising program code instructions of the program according to claim 15.

17. The device according to claim 16, wherein:
- the battery (606), the control unit (608) and the memory (604) are arranged in a case having a bottom forming a surface,
- the electrode (610) is arranged on a flexible material that is substantially in the same plane as the bottom of the case.
